# EUROPÄISCHE PATENTSCHRIFT

(11) **EP 0 947 215 B1**
(45) Veröffentlichungstag und Bekanntmachung des Hinweises auf die Patenterteilung: **16.03.2005**
(21) Anmeldenummer: 99250099.1
(22) Anmeldetag: 31.03.1999
(51) Int. Cl.: A61N 1/365, A61N 1/362

(54) **Vorrichtung zur Aufrechterhaltung eines natürlichen Herzrhythmus**
Device for maintaining the natural cardiac rhythm
Dispositif pour maintenir le rythme cardiaque naturel

(30) Priorität: 31.03.1998 DE 19815539
(43) Veröffentlichungstag der Anmeldung: 06.10.1999
(73) Patentinhaber: BIOTRONIK Mess- und Therapiegeräte GmbH & Co Ingenieurbüro Berlin, 12359 Berlin (DE)
(72) Erfinder: Hastings, David, Lake Oswego, OR 97034 (US); Schaldach, Max, Prof.-Dr., 91054 Erlangen (DE); Rolison, Gary, Portland, OR 97229 (US); Weyant, Robert R., Dureham, OR 97224 (US)
(74) Vertreter: Eisenführ, Speiser & Partner

(56) Entgegenhaltungen:
- WO-A-97/22380
- US-A- 4 917 115
- US-A- 5 584 867
- US-A- 5 628 777

## Beschreibung

Die Erfindung betrifft eine Vorrichtung gemäß dem Oberbegriff des Anspruchs 1.

Aus dem Stand der Technik sind vielfältige Vorrichtungen bekannt, welche mindestens einen innerhalb des Körpers des Patienten angeordneten Sensor zur Aufnahme eines eine Information über den Herzleistungsbedarf enthaltenden körpereigenenen Meßsignals aufweisen, um daraus unter anderem ein Signal für die Beeinflussung der Stimulationsrate herzuleiten.

Die bisherigen Vorrichtungen gingen davon aus, daß eine unmittelbare und zeitgerechte Beziehung zwischen der Veränderung des eine Information über den Herzleistungsbedarf beinhaltenden körpereigenen Meßsignals und der zu beeinflussenden Stimulationsgröße besteht, wie beispielsweise der Amplitude, der Frequenz oder mittleren Ereignishäufigkeit von durch einen Sensor aufgenommenen Signalen und der Herzrate.

Eine Vorrichtung gemäß Oberbegriff von Anspruch 1 ist aus WO-A-97/22 380 bekannt.

Es hat sich jedoch gezeigt, daß eine derartige unmittelbare Verarbeitung im Zeitoder Frequenzbereich häufig nicht zu den gewünschten Ergebnissen führt.

Der Erfindung liegt deshalb die Aufgabe zugrunde, die Steuerung von Stimulationsereignissen und insbesondere der Herzrate zu verbessern.

Die Aufgabe wird, ausgehend von einer Vorrichtung gemäß dem Oberbegriff des Anspruchs 1, durch die im kennzeichnenden Teil des Anspruchs 1 angegebenen Merkmale gelöst.

Die Erfindung schließt die technische Lehre ein, daß bei der Ermittlung einer die Folge des Stimulationssignals beeinflussenden Größe auch solche Zusammenhänge beachtet werden müssen, welche über eine unmittelbaren Zusammenhang hinausgehen. Dazu gehört einerseits eine Transformation der zu berücksichtigenden Zeiträume in der Weise, daß die zeitliche Beobachtung auch über zurückliegende Zeiträume erfolgt und die Steuerung der Herzrate durch den räumlich-zeitlichen Versatz komplexer Frequenz- oder Ereignisfolgen kontrolliert wird, welche Laufzeiten innerhalb des Körpers aufweisen. Diese Laufzeiten werden durch den zeitlichen Versatz von zwei räumlich versetzt angeordneten Meßwertaufnehmern erhalten. Die Übereinstimmung der zugrundeliegenden Ergeignissignale wird dabei bevorzugt durch Mustervergleich verifiziert. Die Laufzeiten der Frequenz- oder Ereignisfolgen werden dabei durch die Kreislaufdynamik und die dieser zugrundeliegerlden körperinternen Regelvorgänge bestimmt.

Auf diese Weise gelingt es, eine körpereigene Dynamik zu "entschlüsseln " und der Herzstimulation zugänglich zu machen, welche bisher zwar schon bei der Steuerung der Vasomotorik des menschlichen Körpers eine wichtige Rolle spielte, aber für die Wiederherstellung des natürlichen Herzthythmus nicht eingesetzt werden konnte.

Bei den auszuwertenden Signalen handelt es sich um Kreislaufgrößen, deren ständiger Wechsel in dynamischen Schwankungen auch eine Information für den Herzleistungsbedarf enthält. Hierbei handelt es sich um Laufzeiten von elektrischen Signalen im Herzen, nämlich die AA-Überleitung, welche bei der Über- bzw. Unterschreitung charakteristischer Werte ein Vorbote für eine drohende Tachykardie bzw. Fibrillation ist. Es sind zu große Laufzeitunterschiede zwischen den beiden Atrien ausreichend früh zu erkennen, um mittels biatrialer Stimulation die Anbahnung einer Tachykardie zu verhindern. Bei Auswertung der entsprechenden Signallaufzeit können also rechtzeitig Gegenmaßnahmen ergriffen werden - dieses sind beispielsweise biatriale Stimulationssequenzen, welche einem entsprechenden Zustand vorbeugen.

Mit der Erfindung ist der wichtige Vorteil verbunden, daß erstmals Signale, welche bisher nicht detektierbar waren oder unbeachtet blieben, die Stimulationsrate mitbestimmen, so daß auf die körperliche Aktivität des Patienten aufnehmende Sensormittel verzichtet werden kann.

Die Anwendung der erfindungsgemäßen Vorrichtung zur Aufrechterhaltung oder Wiederherstellung eines natürlichen Herzrhythmus durch Erzeugung eines elektrischen Stimulationssignals bezieht sich dabei insbesondere auf implantierbare Herzschrittmacher zur Behandlung von Brady- oder Tachykardien sowie entsprechende Herzrhythmuskorrekturgeräte, welche auch als implantierbare Defibrillatoren im Gebrauch sind.

Wenn gemäß der Erfindung den Sensoren eine Verarbeitungseinheit nachgeschaltet ist, welche aus dem Meßsignal ein körpereigenes Laufzeitsignal gewinnt, dessen Dauer einige von einigen Millisekunden bis zu einer Sekunde umfaßt, wobei das körpereigene Laufzeitsignal mindestens mittelbar ein Steuersignal bildet, das den Zeitpunkt und/oder die Zeitfolge oder den Zeitpunkt des Stimulationssignals beeinflußt, so bedeutet dies, daß aus einem dem Kreislaufsystem eingeprägten Laufzeitsignal eine Information entnommen wird, welche für die Herztätigkeit von Bedeutung ist. Es wurde gefunden, daß diese Signale auch für die natürliche Herztätigkeit des Menschen von Bedeutung sind und eine Stimulation in Korrelation zu derartigen Signalen mindestens eine physiologische Ergänzung zu auf andere Weise aus dem Körper des Patienten abgeleiteten, für die Herzfunktion relevanten Signalen bildet.

Die Selektion des körpereigenen Laufzeitsignals kann mittels Digitalfilterung oder der Anwendung einer Korrelationstechnik erfolgen.

Bei einer digitalen Verarbeitung besteht in vorteilhafter Weise auch die Möglichkeit, eine Detektion bzw. Synchronisation der Laufzeitvorgänge durch den Vergleich vollständiger Signalmuster im Amplituden- und/oder Frequenzbereich zu bewirken. Hierbei wird insbesondere in der Verarbeitungseinheit innerhalb eines wenige 100 Millisekunden umfassenden Zeitsegments vorgegebener Dauer mindestens ein periodisch auftretendes Signal oder ein entsprechender Signalanteil mit einem charakteristischen Amplitudenverlaufs- oder Frequenzmuster mittels eines Zeitfensters selektiert und aus der Folge des Auftretens dieses Musters das körpereigene Laufzeitsignal abgeleitet, welches die Zeitfolge oder den Zeitpunkt des Stimulationssignals beeinflußt. Die Breite der bei dieser Verarbeitung zu benutzenden Zeitsegmente entspricht dabei den Laufzeiten der erwarteten Signale oder Signalanteile. Eine auf der Ähnlichkeit oder Übereinstimmung von in den Zeitsegmenten erscheinenden Amplituden- oder Frequenzmustern basierende Ermittlung der Laufzeit beinhaltet den Vorteil, daß eine Ähnlichkeit der die Laufzeitfrequenz bestimmenden Signalanteile auch bei weniger sich wiederholenden Perioden schneller und sicherer zu erkennen ist und nicht das "Einschwingen" von Filterschaltungen oder dergleichen abgewartet werden muß, um die periodischen Signalanteile zu detektieren. Dies ist inbesondere günstig bei größeren Laufzeiten.

Zur Detektion von übereinstimmenden Signalmustern wird dabei insbesondere ein jeweils in dem Zeitsegment aufgenommenes, periodisch auftretendes Signal oder ein entsprechender Signalanteil mit einem charakteristischen Amplitudenverlaufsoder Frequenzmuster gespeichert, wobei das Muster des laufenden Zeitsegments mit mindestens einem gleichartigen, zuvor aufgenommenen, periodisch auftretenden Signal oder einem entsprechenden Signalanteil mit einem charakteristischen Amplitudenverlaufs- oder Frequenzmuster fortlaufend miteinander verglichen wird und in Koinzidenz mit dem später erscheinenden, periodisch auftretenden Signal oder einem entsprechenden Signalanteil mit einem charakteristischen Amplitudenverlaufs- oder Frequenzmuster ein Signal abgegeben wird, wenn der Grad der Übereinstimmung der miteinander zu vergleichenden Muster einen vorgegebenen Wert überschreitet, wobei aus dem die Übereinstimmung anzeigenden Signal die Steuergröße hergeleitet wird, welche die Stimulationsgröße beeinflußt. Anstelle des Frequenzmusters kann dabei auch der zeitliche Verlauf eines oder mehrerer spektraler Anteile in dem Frequenzmuster, welches innerhalb eines Zeitsegments aufgenommen wurde, verglichen werden (Wavelet).

Die Steuergröße, welche die Zeitfolge oder den Zeitpunkt des Stimulationssignals beeinflußt, kann dabei insbesondere auch aus der Rate, der mittleren Häufigkeit, der zeitlichen Änderung der Rate oder dessen mittlerer Häufigkeit des eine Übereinstimmung anzeigenden Signals abgeleitet werden, so daß sich eine statistische Verdichtung der abgeleiteten zur Stimulation herangezogenen Information ergibt. Eine entsprechende Verdichtung der Information läßt sich auch erzielen, wenn die Information aus dem Muster des Amplitudenverlaufs und dem spektralen Muster gemeinsam ausgewertet werden.

Zur Erzeugung von Frequenzspektren wird das zeitabhängige Signal zweckmäßigerweise einer Fast-Fourier-Transformation (FFT) unterworfen.

Als körpereigenes Sensorsignal kommen erfindungsgemäß das herzinterne Laufzeitsignal zwischen den beiden Atrien in Betracht.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

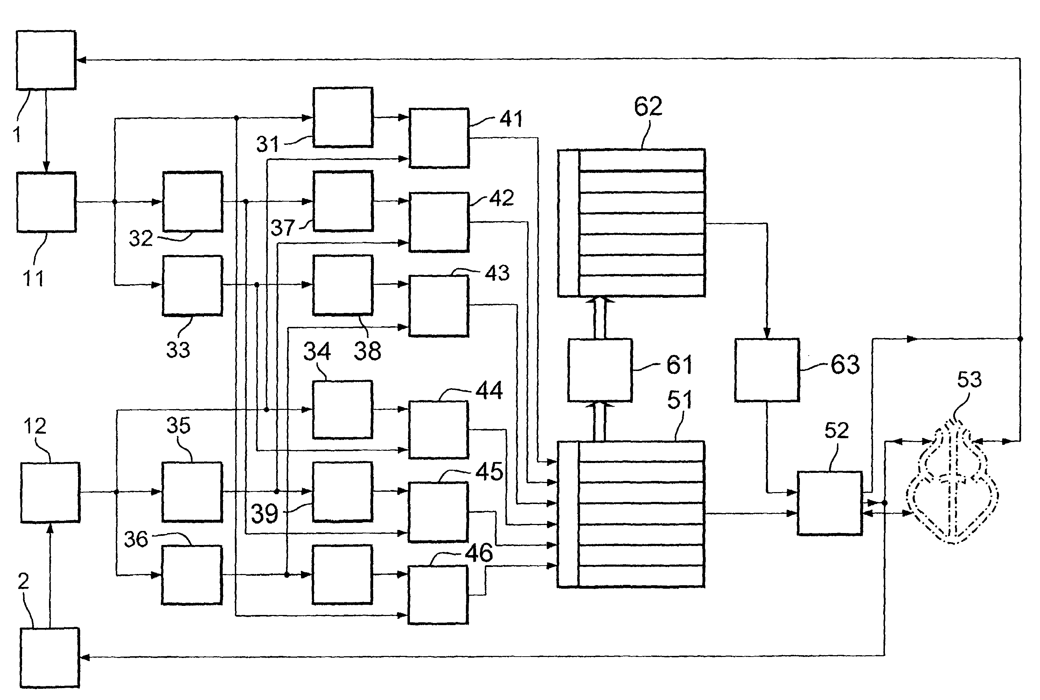

Die einzige Figur zeigt ein bevorzugtes Ausführungsbeispiel der Erfindung im Blockschaltbild.

Bei dem in der Figur dargestellten Ausführungsbeispiel handelt es sich um eine Vorrichtung zur Aufrechterhaltung oder Wiederherstellung eines natürlichen Herzrhythmus durch Erzeugung eines elektrischen Stimulationssignals, in Form eines implantierbaren Herzschrittmachers, welche mindestens einen innerhalb des Körpers des Patienten angeordneten Sensor zur Aufnahme eines eine Information über den Herzleistungsbedarf oder eine andere herzrelevante Größe enthaltenden körpereigenen Signals aufweist. Eine derartige Größe stellt erfindungsgemäß das herzinterne Laufzeitsignal zwischen den beiden Atrien dar.

Dabei ist den Sensoren 1 und 2 eine aus mehreren nachfolgend zu beschreibenden Baugruppen bestehende Verarbeitungseinheit nachgeschaltet, welche aus den diesen Sensoren zugeführten (als Eingangspfeile dargestellten) Meßsignalen ein körpereigenes Laufzeitsignal gewinnt, dessen Dauer mindestens einige Millisekunden umfaßt, wobei das körpereigene Laufzeitsignal mindestens mittelbar ein Steuersignal bildet, das den Zeitpunkt oder die Zeitfolge oder den Zeitpunkt des Stimulationssignals beeinflußt.

Bei den Sensoren 1 und 2 kann es sich dabei um in Bereich der beiden Herzvorhöfe angebrachte Elektroden handeln.

Dazu erfolgt die Selektion des körpereigenen Laufzeitsignals in den Baugruppen im oberen Teil der Figur in einem Frequenzbereich, der im wesentlichen dem Tag/Nacht-Rhythmus - und damit der inneren Uhr des Menschen - entspricht. Im unteren Teil der Figur erfolgt hingegen eine Selektion des körpereigenen Laufzeitsignals in einem Frequenzbereich, der im wesentlichen einem 6 bis 20 Sekunden-Rhythmus, insbesondere 10 Sekunden-Rhythmus, entspricht. Er entspricht damit einem Bruchteil der Atemfrequenz.

In der Zeichnung sind in den Blöcken 1 1 und 12 Verstärkerschaltungen für die von den Sensoren aufgenommenen Signale vorgesehen. In Speicherbausteinen wird in Blöcken 31 und 34 werden jeweils der über ein Zeitsegment des Sensorsignals ermittelte Amplitudenverlauf gespeichert. In den Blöcken 41 und 44 erfolgt für die jeweiligen Signale innerhalb vorgegebener, an die Signallaufzeit angepaßter Zeitfenster eine Ermittlung des zeitlichen Versatzes des späteren Signals zu dem zuerst aufgenommen A-Ereignis der jeweils anderen Kammer.

Bei der Darstellung in der Figur wird die Laufzeit der beiden A-A-Ereignisse relativ zu einander in zwei Verarbeitungszweigen (oberer und unter Teil) ermittelt. Dabei ergeben sich also bei zyklischem Verlauf zwei Laufzeiten, wobei jedes der A-Ereignisse in den beiden Kammern einmal als das vorangehende angesehen wird.

Die Feststellung der Übereinstimmung des Signals mit dem erwarteten erfolgt durch Muster- oder Ähnlichkeitsvergleich. Bei festgestellter Übereinstimmung wird der Zeitabstand ermittelt und dieser als für die Dynamik charakteristisches Signal weiterverarbeitet.

In den Stufen 32 und 35 wird zusätzlich eine Fast-Fourier-Analyse vorgenommen, so daß als Signalmuster für den Vergleich des aktuell erscheinenden mit einem Verzögerungsglied 37 bzw. 39 zeitlich verzögerten Signal in den Stufen 42 bzw. 45 das mittlere Frequenzspektrum in dem jeweiligen Zeitsegment dient.

In den Stufen 33 und 36 werden statt dessen Wavelets aus dem laufenden Eingangssignal erzeugt und - nach Verzögerung in Verzögerungsgliedern 37 bzw. 39 - in den Stufen 43 und 46 ebenfalls miteinander verglichen, so daß aus der Wiederholung entsprechender Wavelets ebenfalls auf eine Periodizität des entsprechenden Sensorsignals geschlossen werden kann.

Hierbei wird in der Verarbeitungseinheit innerhalb eines mindestens mehrere Millisekunden umfassenden Zeitsegments vorgegebener Dauer mindestens ein periodisch auftretendes Signal oder ein entsprechender Signalanteil mit einem charakteristischen Amplitudenverlaufs- bzw. Frequenzmuster selektiert und festgehalten.

Das jeweils in dem Zeitsegment aufgenommene, periodisch auftretende Signal oder ein entsprechender Signalanteil mit einem charakteristischen Amplitudenverlaufsoder Frequenzmuster wird in den nachfolgenden Stufen gespeichert, das laufende Zeitsegment mit mindestens einem gleichartigen, zuvor aufgenommenen, periodisch auftretenden Signal oder ein entsprechender Signalanteil mit einem charakteristischen Amplitudenverlaufs- oder Frequenzmuster fortlaufend miteinander verglichen und in Koinzidenz mit dem später erscheinenden, periodisch auftretenden Signal oder einem entsprechenden Signalanteil mit einem charakteristischen Amplitudenverlaufs- oder Frequenzmuster ein Signal abgegeben, wenn der Grad der Übereinstimmung der miteinander zu vergleichenden Muster einen vorgegebenen Wert überschreitet. Die Periodizität der dynamischen Signale ergibt sich damit aus der Signalverzögerung bei aufeinanderfolgenden Signalen, welche notwendig ist, diese Signale (sei es der Amplitudenverlauf oder das Spektrum) mit ihrem jeweiligen Vorgänger nach einem Ähnlichkeits- oder Korrelationsverfahren "zur Deckung" zu bringen.

Die Ausgangssignale der Stufen 41 bis 46 stehen damit für die jeweils durch die Art der Verarbeitungsmittel 31 bis 46 bestimmten Laufzeiten der von den Sensoren 1 und 2 in den Eingangssignalen detektierten dynamischen Signalanteile. Durch Adressierung eines nachfolgenden als Nachschlagetabelle ausgebildeten Speichers erfolgt die Kombination der für die Laufzeiten der Signale repräsentativen Ausgangssignale der Stufen 41 bis 46 als Adressierungssignale für den Speicher, der seinerseits die an den adressierten Speicherplätzen abgelegten Steuersequenzen an den konventionellen Herzstimulator oder Defibrillator abgibt. Dabei kann es sich um ein Ratensteuersignal oder um ein singuläres Signal zur Auslösung einer einzelnen Tachykardie-Terminierungs- oder Defibrillationssequenz handeln.

Es ist ersichtlich, daß durch Kombination der Ausgangssignale der Stufen 41 bis 46 auch komplexere Auswertungen der körperdynamischen Laufzeitsignale vorgenommen werden können. Hierzu kommen insbesondere deren logische Verküpfungen, Überlagerungen, zeitliche Ableitungen, Häufungswerte etc. in Betracht.

Die Inhalte der adressierten Speicherplätze des Tabellenspeichers 51 geben ein Ausgangssignal an den - im übrigen konventionellen steuerbaren Stimulatorteil 52 ab, der in bekannter Weise in Wechselwirkung mit dem Herzen 53 steht. Durch die Steuersignale vom Ausgang des Speichers 51 wird im Falle eines ratengesteuerten Schrittmachers die Grundrate beeinflußt, während im Falle eines Defibrillators ein Defibrillationszyklus ausgeführt wird, wenn Körperlaufzeitsignale einen vorgegebenen Schwellwert überschreiten.

Da es sich bei der Vorrichtung um eine solche handelt, bei der eine körpereigene Laufzeitgröße welche die Herzstimulation beeinflußt, durch die körperinterne Kopplung selbst wieder beeinflußt werden kann, kann - wie bei jedem Regelsystem - die Gefahr einer Schwingneigung gegeben sein. Zur Vermeidung sind deshalb Mittel vorgesehen, welche bei einer Überhöhung der mittleren Amplitude einer für die Kreislaufdynamik charakteristischen Größe entsprechende Gegenmaßnahmen einleiten. Hierzu wird von den Stufen 41 bis 46 nicht nur der für die jeweilige innere Kreislaufdynamik charakteristische Zeitabstand der aufeinanderfolgenden Mustersignale, sondern auch deren mittlere Amplitude zur Adressierung des Tabellenspeichers 51 benutzt. Überschreitet diese Amplitude einen vorgegeben Maximalwert, so wird über eine Schaltstufe 61 ein weiterer Tabellenspeicher 62 adressiert, welcher über eine Ausgangsstufe 63 eine Änderung der Stimulationsgröße im Verhältnis zum Verlauf der zeitlichen Änderung der ermittelten Körperlaufzeitgröße um einen vorgegebenen oder einen statistisch ermittelten Verzögerungswert bewirkt.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch machen.

## Patentansprüche

1. Vorrichtung zur Aufrechterhaltung oder Wiederherstellung eines natürlichen Herzrhythmus durch Erzeugung eines elektrischen Stimulationssignals, insbesondere in Form eines implantierbaren Herzschrittmachers, welche zwei innerhalb des Körpers des Patienten angeordnete Sensoren zur Aufnahme eines eine Information über den Herzleistungsbedarf oder eine andere herzrelevante Größe enthaltenden körpereigenen Signals aufweist, wobei den Sensoren eine Verarbeitungseinheit nachgeschaltet ist, welche aus der zeitlichen Differenz eines bei den beiden Sensoren nacheinander einlaufenden körpereigenen Signals ein körpereigenes Laufzeitsignal gewinnt, welches repräsentativ für die Dauer der ermittelten Laufzeit ist, wobei das körpereigene Laufzeitsignal mindestens mittelbar ein Steuersignal bildet, das den Zeitpunkt und/oder die Zeitfolge des Stimulationssignals beeinflußt, **dadurch gekennzeichnet, daß** es sich bei dem körpereigenen Laufzeitsignal um den zeitlichen Versatz der atrialen Ereignisse in den beiden Herzkammern handelt.

2. Vorrichtung nach Anspruch 1, **dadurch gekennzeichnet, daß** die Selektion des körpereigenen Laufzeitsignals mittels Digitalfilterung oder der Anwendung einer Korrelationstechnik erfolgt.

3. Vorrichtung nach Anspruch 1, **dadurch gekennzeichnet, daß** in der Verarbeitungseinheit innerhalb eines wenige 100 Millisekunden umfassenden Zeitsegments vorgegebener Dauer mindestens ein periodisch auftretendes Signal oder ein entsprechender Signalanteil mit einem charakteristischen Amplitudenverlaufs- oder Frequenzmuster selektiert und aus der Aufeinanderfolge des Auftretens dieses Musters das körpereigene Laufzeitsignal hergeleitet wird, welches die Zeitfolge und/oder den Zeitpunkt des Stimulationssignals beeinflußt, wobei die aufeinanderfolgenden Zeitsegmente einen vorgegebenen Zeitabstand aufweisen, welcher der erwarteten Dauer der Laufzeit entspricht.

4. Vorrichtung nach Anspruch 3, **dadurch gekennzeichnet, daß** das jeweils in dem Zeitsegment aufgenommene, zeitversetzt auftretende Signal oder der entsprechende Signalanteil mit einem charakteristischen Amplitudenverlaufsoder Frequenzmuster gespeichert, das laufende Zeitsegment mit mindestens einem gleichartigen, zuvor aufgenommenen, zeitversetzt auftretenden Signal oder ein entsprechender Signalanteil mit einem charakteristischen Amplitudenverlaufs- oder Frequenzmuster verglichen und bei Koinzidenz mit dem später erscheinenden, ein die Zeitdifferenz des Erscheinens anzeigendes Signal abgegeben wird, wenn der Grad der Übereinstimmung der miteinander zu vergleichenden Muster einen vorgegebenen Wert überschreitet, wobei aus dem die Zeitdifferenz anzeigenden Signal das körpereigene Laufzeitsignal erzeugt wird.

5. Vorrichtung nach einem der vorhergehenden Ansprüche, **dadurch gekennzeichnet, daß** anstelle des Frequenzmusters der zeitliche Verlauf eines oder mehrerer spektraler Anteile in dem Frequenzmuster verglichen werden.

6. Vorrichtung nach einem der Ansprüche 3 - 5, **dadurch gekennzeichnet, daß** das körpereigene Laufzeitsignal aus der Überlagerung oder Kumulation mehrerer aus der Rate, der mittleren Häufigkeit, der zeitlichen Änderung der Rate oder dessen mittlerer Häufigkeit des eine Übereinstimmung anzeigenden Signals für mindestens zwei Zeitsegmente unterschiedlicher Dauer erzeugt wird.

7. Vorrichtung nach einem der Ansprüche 3 - 6, **dadurch gekennzeichnet, daß** die Steuergröße, welche die Zeitfolge und/oder den Zeitpunkt des Stimulationssignals beeinflußt, durch Überlagerung oder Kumulation mehrerer aus der Rate, der mittleren Häufigkeit, der zeitlichen Änderung der Rate oder dessen mittlerer Häufigkeit des eine Übereinstimmung anzeigenden Signals für mindestens zwei Zeitsegmente gleicher Dauer erzeugt wird, bei denen einerseits der Ampütudenverlauf und andererseits der Verlauf des Frequenzmusters ausgewertet wird.

8. Vorrichtung nach einem der vorhergehenden Ansprüche, **dadurch gekennzeichnet, daß** das Frequenzmuster einer Fast-Fourier-Transformation (FFT) unterworfen wird.

9. Vorrichtung nach einem der vorhergehenden Ansprüche, **dadurch gekennzeichnet, daß** es sich bei dem Steuersignal, welches die Zeitfolge oder den Zeitpunkt des Stimulationssignals beeinflußt, um die Stimulationsrate oder ein Tachykardieterminierungs- bzw. Defibrillationssignal handelt.

10. Vorrichtung nach einem der vorhergehenden Ansprüche, **dadurch gekennzeichnet, daß** Mittel vorgesehen sind, welche bei Feststellung einer Amplitudenüberhöhung des aktuellen zeitlichen Mittelwertes des körpereigenen Laufzeitsignals im Vergleich zu einem vorangehenden Langzeitmittelwert die Phasenlage der Änderung des Stimulationsgröße im Verhältnis zum Verlauf der zeitlichen Änderung der ermittelten Laufzeitgröße um einen vorgegebenen oder einen statistisch ermittelten Größe verändern.

11. Vorrichtung nach einem der vorhergehenden Ansprüche, **dadurch gekennzeichnet, daß** Vergleiche von Mustern, Spektren oder Signalverläufen in der Verarbeitungseinheit nach der Methode der kleinsten Fehlerquadrate ausgeführt werden.

12. Vorrichtung nach einem der vorhergehenden Ansprüche, **dadurch gekennzeichnet, daß** bei Überschreitung eines vorgegebenen zeitlichen Versatzes zwischen den atrialen Ereignissen der beiden Herzkammern eine Stimulationssequenz zur präventiven Tachykardie- oder Fibrillationsbehandlung ausgelöst wird.

## Claims

1. Device for maintaining or re-establishing a natural heart rhythm by generating an electrical stimulation signal, in particular in the form of an implantable pacemaker, which comprises two sensors arranged inside the patient's body for picking up a body-specific signal containing information regarding the heart performance requirement or another variable that is relevant to the patient's heart, a processing unit, which obtains a body-specific time-delay signal, which is representative of the duration of the ascertained time delay, from the temporal difference of a body-specific signal arriving in succession at the two sensors, being connected downstream of the sensors, the body-specific time-delay signal forming, at least indirectly, a control signal that influences the point in time and/or the time sequence of the stimulation signal, **characterised in that** the body-specific time-delay signal relates to the time-displacement of the atrial events in the two ventricles.

2. Device according to claim 1, **characterised in that** the body-specific time-delay signal is selected by means of digital filtering or by the use of a correlation technique.

3. Device according to claim 1, **characterised in that** at least one periodically appearing signal or a corresponding signal portion having a characteristic amplitude curve pattern or frequency pattern is selected in the processing unit within a time segment of a predetermined duration and comprising less than 100 milliseconds, and the body-specific time-delay signal, which influences the time sequence and/or the point in time of the stimulation signal, is derived from the succession of the appearance of this pattern, the sequential time segments displaying a predetermined interval that corresponds to the expected duration of the time delay.

4. Device according to claim 3, **characterised in that** the signal picked up in each case in the time segment and appearing in a time-displaced manner or the corresponding signal portion having a characteristic amplitude curve pattern or frequency pattern is stored, the current time segment is compared with at least one similar, previously picked-up signal appearing in a time-displaced manner or a corresponding signal proportion having a characteristic amplitude curve pattern or frequency pattern and, in the event of coincidence with the later-appearing signal, a signal indicating the time difference of the appearance is issued if the degree of correspondence of the patterns to be compared with one another exceeds a predetermined value, the body-specific time-delay signal being generated from the signal indicating the time difference.

5. Device according to any one of the preceding claims, **characterised in that**, instead of the frequency pattern, the time characteristics of one or more spectral portions in the frequency pattern are compared.

6. Device according to any one of claims 3 to 5, **characterised in that** the body-specific time-delay signal is generated from the superposition or accumulation of a plurality of the following: the rate, the average frequency, the temporal change of the rate or the average frequency thereof of a signal indicating correspondence, for at least two time segments of different durations.

7. Device according to any one of claims 3 to 6, **characterised in that** the control variable that influences the time sequence and/or the point in time of the stimulation signal is generated by the superposition or accumulation of a plurality of the following: the rate, the average frequency, the temporal change of the rate or the average frequency thereof of a signal indicating correspondence, for at least two time segments of the same duration, wherein the amplitude curve, on the one hand, and the characteristic of the frequency pattern, on the other hand, are evaluated.

8. Device according to any one of the preceding claims, **characterised in that** the frequency pattern is subjected to a Fast-Fourier-Transformation (FFT).

9. Device according to any one of the preceding claims, **characterised in that** the control signal that influences the point in time and/or the time sequence of the stimulation signal is the stimulation rate or a tachycardia termination signal or defibrillation signal.

10. Device according to any one of the preceding claims, **characterised in that** means are provided that, on discovering an excessive rise in amplitude of the current temporal average value of the body-specific time-delay signal, in comparison to a preceding long-term average value, alter the phase position of the change of the stimulation variable relative to the characteristic of the temporal change of the determined time-delay variable, by a predetermined or a statistically-determined variable.

11. Device according to any one of the preceding claims, **characterised in that** patterns, spectra or signal characteristics are compared in the processing unit using the smallest-error-square method.

12. Device according to any one of the preceding claims, **characterised in that**, when a predetermined time-displacement between the atrial events of the two ventricles is exceeded, a stimulation sequence is triggered for the purpose of preventive tachycardia treatment or fibrillation treatment.

## Revendications

1. Dispositif pour le maintien ou le rétablissement d'un rythme cardiaque naturel moyennant la production d'un signal de stimulation électrique, notamment sous la forme d'un simulateur cardiaque implantable, qui comporte deux capteurs disposés à l'intérieur du corps du patient, servant à recevoir un signal propre au corps, contenant une information concernant le besoin de puissance pour le coeur ou une autre grandeur importante pour le coeur, dans lequel en aval des capteurs est branchée une unité de traitement, qui détermine, à partir de la différence d'un signal propre au corps, qui arrive successivement au niveau des deux capteurs, un signal de temps de propagation propre au corps, qui est représentatif de la durée du temps de propagation déterminé, et dans lequel le signal de temps de propagation propre au corps forme au moins indirectement un signal de commande qui inclut sur l'instant et/ou la séquence temporelle du signal de stimulation, **caractérisé en ce qu'**en ce qui concerne le signal de propagation propre au corps, il s'agit du décalage temporel des phénomènes atriaux dans les deux cavités du coeur.

2. Dispositif selon la revendication 1, **caractérisé en ce que** la sélection du signal du temps de propagation propre au corps est réalisée à l'aide d'un filtrage numérique ou de l'utilisation d'une technique de corrélation.

3. Dispositif selon la revendication 1, **caractérisé en ce que** dans l'unité de traitement, au cours d'un segment temporel d'une durée prédéterminée comprenant quelques centaines de millisecondes, au moins un signal apparaissant périodiquement ou une composante correspondante de signal ayant un modèle caractéristique de variation d'amplitude ou de fréquence est sélectionné et, qu'à partir de la succession de l'apparition de ce modèle est déterminé le signal de temps de propagation propre au corps, qui influe sur la séquence temporelle et/ou l'instant du signal de stimulation, les segments temporels successifs étant séparés par un intervalle de temps prédéterminé, qui correspond à la durée attendue du temps de propagation.

4. Dispositif selon la revendication 3, **caractérisé en ce que** le signal, qui est capté respectivement dans le segment temporel et apparaît d'une manière décalée dans le temps, ou la composante correspondante du signal comportant un modèle caractéristique d'allure d'amplitude ou de fréquence est mémorisée que le segment temporel en cours important est comparé à au moins un signal de même type, capté auparavant et apparaissant d'une manière décalée dans le temps ou une composante de signal correspondante comportant un modèle caractéristique d'allure d'amplitude ou de fréquence et, en cas de coïncidence avec le signal apparaissant ultérieurement, un signal indiquant la différence de temps d'apparition est délivré, lorsque le degré de concordance des modèles devant être comparés entre eux dépasse un modèle prédéterminé, auquel cas le signal de temps de propagation propre au corps est produit à partir du signal indiquant la différence de temps.

5. Dispositif selon l'une des revendications précédentes, **caractérisé en ce qu'**à la place du modèle de fréquence, on compare les variations dans le temps d'une ou de plusieurs composantes spectrales dans le modèle de fréquence.

6. Dispositif selon, l'une des revendications 3 à 5, **caractérisé en ce que** le signal de temps de propagation propre au corps est produit dans le cas de la superposition ou du cumul de plusieurs éléments parmi le rythme, la fréquence moyenne, la variation dans le temps du rythme ou sa fréquence moyenne d'un signal indiquant une concordance, pour au moins deux segments temporels ayant des durées différentes.

7. Dispositif selon l'une des revendications 3 à 6, **caractérisé en ce que** la grandeur de commande, qui influe sur la séquence temporelle et/ou l'instant du signal de stimulation, est produite par superposition ou cumul de plusieurs des éléments comprenant le rythme, la fréquence moyenne, la variation dans le temps du rythme ou de sa fréquence moyenne du signal indiquant une concordance, sur au moins deux segments temporels de même durée, dans lesquels d'une part la variation d'amplitude et d'autre part la variation du modèle de fréquence sont évaluées.

8. Dispositif selon l'une des revendications précédentes, **caractérisé en ce que** le modèle de fréquence est soumis à une transmission de Fourier rapide (FFT).

9. Dispositif selon l'une des revendications précédentes, **caractérisé en ce qu'**en ce qui concerne le signal de commande, qui influe sur la séquence temporelle ou sur l'instant du signal de stimulation, il s'agit de la cadence de stimulation ou d'un signal de détermination de tachycardie ou d'un signal de défibrillation.

10. Dispositif selon l'une des revendications précédentes, **caractérisé en ce qu'**il est prévu les moyens qui, lors de la détermination d'un accroissement de l'amplitude de la valeur moyenne temporelle actuelle du signal de propagation propre au corps, par rapport à la valeur moyenne précédente de longue durée, modifient la position de phase de la variation de la grandeur de stimulation par rapport à l'allure de la variation dans le temps de la grandeur déterminée du temps de propagation et ce d'une grandeur prédéterminée ou d'une grandeur déterminée de façon statistique.

11. Dispositif selon l'une des revendications précédentes, **caractérisé en ce que** des comparaisons de modèles, de spectres ou de variations de signaux sont exécutées dans l'unité de traitement selon la méthode des moindres carrés.

12. Dispositif selon l'une des revendications précédentes, **caractérisé en ce que** dans le cas du dépassement d'un décalage temporel prédéterminé entre les évènements atriaux des deux cavités du coeur, une séquence de stimulation est déclenchée pour un traitement préventif d'une tachycardie ou d'une fibrillation.
